# EUROPEAN PATENT APPLICATION

(11) **EP 3 964 840 A1**
(43) Date of publication of application: **09.03.2022**
(21) Application number: 20810730.0
(22) Date of filing: 12.05.2020
(51) Int. Cl.: G01N 37/00, C12M 1/00, C12M 1/34

(54) **MICROCHIP**

(30) Priority: 23.05.2019 JP 2019096988
(71) Applicant: Ushio Denki Kabushiki Kaisha, Tokyo 100-8150 (JP)
(72) Inventor: NOMOTO,Daisuke, Tokyo 100-8150 (JP)
(74) Representative: Tomerius, Isabel
(86) International application number: PCT/JP2020/019012
(87) International publication number: WO 2020/235398

(57) **Abstract**

Providing a microchip which is capable of being manufactured with simple equipment, and improving the bonding strength between the first substrate and the second substrate while forming the desired microstructure therein. A microchip includes: a first substrate 10; a second substrate 20 that is partially bonded to the first substrate, the second substrate having a main surface and an outer side face; a hollow channel 23 that is located between the first substrate and the second substrate, the channel 23 extending in a direction along the main surface of the second substrate; a liquid distribution port (21, 22) that is formed to penetrate the second substrate from the channel toward the main surface thereof located at an opposite side of the first substrate; a first bonding section 31 that bonds the first substrate to the second substrate to surround the channel when viewed from a direction orthogonal to the main surface; a second bonding section 32 located at a position closer to the outer side face 24 of the second substrate than the first bonding section, and that bonds the first substrate to the second substrate; and an internal space 25 provided between the first bonding section and the second bonding section, and that communicates with a space outside the first substrate and the second substrate.

## Description

### TECHNICAL FIELD

The present invention relates to a microchip and more particularly a microchip in which a plurality of substrates is bonded and that has a liquid distribution port and a hollow channel that communicates with the liquid distribution port.

### BACKGROUND ART

Conventionally, culturing cells or tissue is performed using a culture dish or a culture plate on which a culture medium such as agar is applied. Since culturing cells or tissue using the culture dish or the culture plate is performed in a two-dimensional (planar) environment, it is difficult to reproduce extracellular microenvironment. In recent years, a microchip (also referred to a biochip) having a microchannel capable of achieving a three-dimensional (stereoscopic) environment for culturing cells or tissue has been proposed.

Patent Document 1 described below discloses an example of a microchip that is capable of culturing cells or tissue.

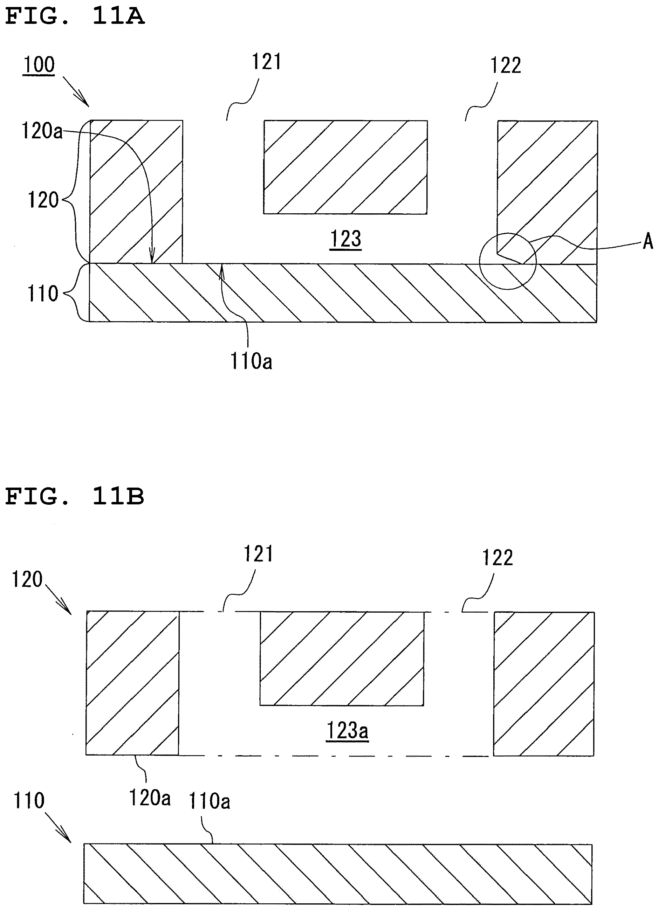

FIG. 11A is a cross-sectional view schematically illustrating a microchip 100 (referred to "resin structure" in Patent Document 1) disclosed in Patent Document 1. The microchip 100 is formed by stacking a second substrate 120 on a first substrate 110 and bonding to their main surfaces that are in contact with each other.

FIG. 11B is a cross-sectional view schematically illustrating the two substrates just before bonding the second substrate 120 to the first substrate 110 in the manufacturing process of the microchip 100. The first substrate 110 and the second substrate 120 each are in the form of a plate. The second substrate 120 has a liquid distribution port (121, 122) for injecting or discharging a culture medium containing cells and a recess 123a that communicates with the liquid distribution port.

The bonding of the first substrate 110 and the second substrate 120 is performed as follows. First, polymer material is applied to one main surface 110a of the first substrate 110 to form a thin film made of polymer material (not shown) using a spin coating method. After that, one main surface 120a of the second substrate 120 is stacked to be in contact with the main surface 110a on which the thin film has been applied, and is pressed under a heated environment. The thin film serves as an adhesive for bonding the first substrate 110 and the second substrate 120.

By bonding the first substrate 110 and the second substrate 120 together, the recess 123a acts as a hollow channel 123 sandwiched between the two substrates (110, 120). When culture medium containing cells is injected through the liquid distribution ports (121, 122), the hollow channel 123 can be used as a place to culture cells.

Among the area of the main surface 120a of the second substrate 120 facing the first substrate 110, all of the area except an area where the recess 123a is formed are in contact with the main surface 110a of the first substrate 110 and bonded through the thin film.

### CITATION LIST

### PATENT DOCUMENT

Patent Document 1: JP-A-2001-038811

### SUMMARY OF INVENTION

### Technical Problem

Since the first substrate 110 and the second substrate 120 are in contact with each other in all of the area except the area where the recess 123a is formed, the area (bonding area) where the second substrate 120 is in contact with the first substrate 110 and pressed thereto is large. As a result, the pressing force applied to both of the substrates is spread out when bonding the first substrate 110 to the second substrate 120. Hence, the pressing force per unit bonding area is reduced, and a portion with weak bonding strength may appear on the microchip after the bonding. In particular, when a portion of bonding sections having the weak bonding strength occurs adjacent to the channel 123, the bonding may partially delaminate, as shown in part A of FIG. 11A.

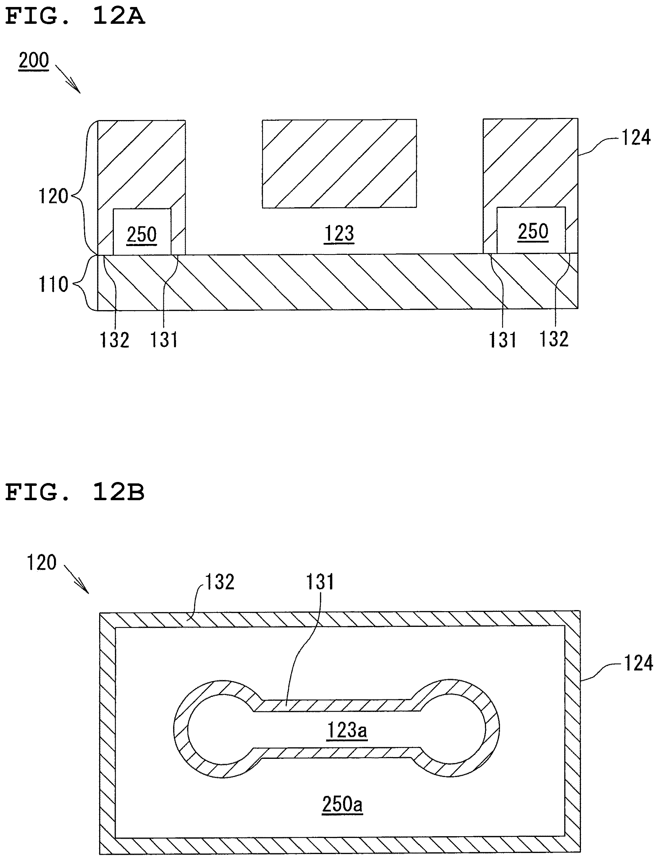

Hence, the present applicant proposed the microchip 200 shown in FIG. 12A. FIG. 12B is a plan view of the second substrate 120 used in the microchip 200 when viewed from the main surface that is to be bonded to the first substrate 110, the second substrate 120 being in the state before bonding to the first substrate 110. The microchip 200 has an internal space 250 in the area surrounded by bonding sections of the second substrate 120 that are bonded to the first substrate 110. The internal space 250 is formed by implementing a recess 250a (see FIG. 12B) in the center of the area that was initially the bonding surface of the second substrate 120.

The internal space 250 divides the bonding sections between the two substrates (110, 120) into a first bonding section 131 adjacent to and surrounding the channel 123, and a second bonding section 132 located on a place closer to the outer surface 124 of the second substrate 120 than the first bonding area 131. The two substrates (110, 120) have a smaller bonding area compared with the case in which the second substrate 120 has no internal space 250. Under the conditions of the same pressing force, the pressing force per unit bonding area increases when the bonding area decreases. Hence this configuration increases the bonding strength of the first bonding section 131, preventing liquid component from invading the first bonding section 131.

However, the inventor's intensive research has revealed that a microchip 200 having the internal space 250 in the area surrounded by the bonding section as shown in FIG. 12a may pose the following problems.

In the microchip 200, the internal space 250 is sealed from the surroundings. During the bonding of the first substrate 110 and the second substrate 120, the air in the internal space 250 thermally expands due to heating during the bonding process, thus the air acts to separate the two substrates (110, 120), thereby reducing the bonding strength of the two substrates (110, 120).

When the microchip 200 returns to a room temperature after the bonding, the air inside the internal space 250, which was previously thermally expanded, cools down to cause the internal space 250 to become negative pressure compared to the external space. Hence, the bonding sections (131, 132) that defines the internal space 250 is subjected to the pressing force caused by the pressure difference from the external space. When subjected to the pressing force, the liquid component in the channel may easily invade the first bonding section 131 adjacent to the internal space 250, which is under negative pressure. If the culture medium containing cells or tissue leaks outside the internal space 250, unintended culture environment may be obtained, which is undesirable.

In order to achieve the pressure in the internal space 250 same as that in the external space, a method of heating and bonding both substrates (110, 120) in a reduced pressure environment is considered; however, this method involves additional vacuum equipment for the reduced pressure environment. If the pressure is reduced excessively, there may remain a pressure difference from the external space.

The present invention is made in consideration of the above circumstances, and it is an object of the present invention to provide a microchip which is capable of being manufactured with simple equipment, and improving the bonding strength between the first substrate and the second substrate while forming the desired microstructure therein.

### Solution to the Problem

The microchip according to the present invention includes:
a first substrate;
a second substrate that is partially bonded to the first substrate, the second substrate having a main surface and an outer side face;
a hollow channel that is located between the first substrate and the second substrate, the channel extending in a direction along the main surface of the second substrate;
a liquid distribution port that is formed to penetrate the second substrate from the channel toward the main surface thereof located at an opposite side of the first substrate;
a first bonding section that bonds the first substrate to the second substrate to surround the channel when viewed from a direction orthogonal to the main surface;
a second bonding section located at a position closer to the outer side face of the second substrate than the first bonding section, and that bonds the first substrate to the second substrate; and
an internal space provided between the first bonding section and the second bonding section, and the internal space communicating with a space outside the first substrate and the second substrate.

The above configuration has the internal space that communicates a space outside the first substrate and the second substrate between the first bonding section and the second bonding section. Hence, even when the first substrate and the second substrate is heated during bonding, the problem in which the air in the internal space expands significantly at the microchip 200 shown in FIG. 12A does not occur, because the internal space maintains an atmospheric pressure substantially same as that of an external space. Therefore, the bonding strength between the first substrate and the second substrate increases, compared with that of the microchip 200 shown in FIG. 12A.

In addition, since the internal space communicates with the external space, the internal space does not likely to have a negative pressure even when cooled down after the bonding. As a result, the case in which the culture medium stored in the channel leaks into the side of the first bonding section is not likely to occur.

Moreover, since the above microchip is provided with the internal space between the first bonding section and the second bonding section and the internal space communicates with the external space, the pressure of the internal space can be set to be substantially same as that of the external space, thus no reduced pressure environment is additionally necessary during the bonding. Hence, the above microchip is capable of being manufactured with simple equipment. This internal space is formed by forming recesses at predetermined locations on the first substrate and/or second substrate in advance and bonding them together.

In addition, the second bonding section is characterized by including a portion that has a line shape connecting a starting point with an end point that is different from the line when viewed from a direction orthogonal to the main surface.

Furthermore, the second bonding section is characterized by including a bonding section that has a polyline shape connecting a starting point with an end point that is different from the starting point, and the bonding section extending with being in contact with the outer side face of the second substrate when viewed from a direction orthogonal to the main surface. This configuration enables a stable bonding of both of the substrates.

Yet furthermore, the second bonding section is characterized by including a plurality of line segment bonding sections that are composed of line segments extending along the outer side faces of the second substrate without being in contact with the outer side faces thereof when viewed from a direction orthogonal to the main surface, the line segment bonding sections being formed apart from each other.

Yet furthermore, the second bonding section is characterized by including a spiral bonding section that is formed such that its center is located at the first bonding section when viewed from a direction orthogonal to the main surface.

The second bonding section is characterized by including local bonding sections that are disposed discretely at a plurality of locations when viewed from a direction orthogonal to the main surface.

In addition, the local bonding sections each are characterized by a circular shape, an elliptical shape or a polygonal shape when viewed from a direction orthogonal to the main surface.

The second bonding section is characterized in that a part of the second bonding section is connected to the first bonding section when viewed from a direction orthogonal to the main surface.

### ADVANTAGE EFFECTS OF THE INVENTION

The present invention is capable of providing a microchip that is manufactured with simple equipment, and improves the bonding strength between the first substrate and the second substrate while forming a desired microstructure therein.

### BRIEF DESCRIPTION OF DRAWINGS

FIG. 1 is a perspective view illustrating a first substrate and a second substrate shortly before bonding the substrates.
FIG. 2 is a plan view of the second substrate before bonding according to a first embodiment.
Fig. 3 is a schematic cross-sectional view of a microchip taken along line B-B of the second substrate in FIG. 2.
Fig. 4 is a schematic cross-sectional view of the microchip taken along line C-C of the second substrate in FIG. 2.
Fig. 5 is a schematic cross-sectional view of the first substrate and the second substrate shortly before bonding the substrates.
FIG. 6 is a plan view of a second substrate before bonding according to a second embodiment.
Fig. 7 is a schematic cross-sectional view of a microchip taken along line E-E of the second substrate in FIG. 6.
FIG. 8 is a plan view of a second substrate before bonding according to a third embodiment.
FIG. 9A is a plan view of a second substrate before bonding according to a fourth embodiment.
FIG. 9B is a plan view of a second substrate before bonding according to another example of the fourth embodiment.
FIG. 10A is a plan view of a second substrate before bonding according to a fifth embodiment.
FIG. 10B is a plan view of a second substrate of a microchip before bonding according to another example of the fifth embodiment.
FIG. 11A is a schematic cross-sectional view of a conventional microchip.
Fig. 11B is a schematic cross-sectional view of a first substrate and a second substrate shortly before bonding the substrates in the manufacturing process of the conventional microchip.
FIG. 12A is a schematic cross-sectional view of a microchip provided with a sealed space between a first bonding section and a second bonding section.
FIG. 12B is a plan view of a second substrate provided with a sealed space between a first bonding section and a second bonding section.

### DESCRIPTION OF EMBODIMENTS

A microchip according to the present invention will now be described with reference to the drawings. It is noted that each drawing disclosed in the present specification is merely schematically illustrated. In other words, the dimensional ratios on the drawings do not necessarily match the actual dimensional ratios, and the dimensional ratios between each drawing do not necessarily match either.

### [First Embodiment]

A first embodiment of a microchip according to the present invention will be described.

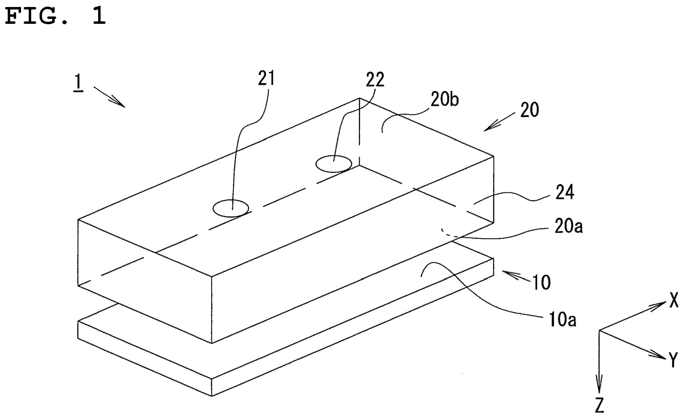

FIG. 1 is a perspective view illustrating a state of a microchip 1 according to the present embodiment before manufacturing. More specifically, the microchip 1 includes a first substrate 10 and a second substrate 20 and is manufactured to bond the substrates as described later. FIG. 1 corresponds to a perspective view illustrating the substrates before bonding the first substrate 10 to the second substrate 20.

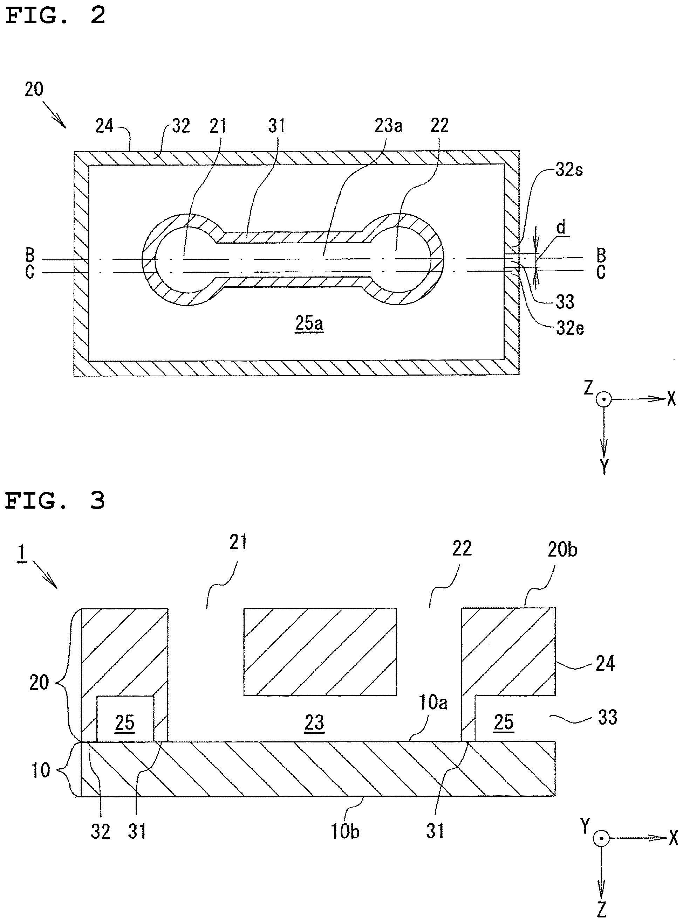

The microchip 1 is formed by stacking one main surface 20a of the second substrate 20 on one main surface 10a of the first substrate 10 so as to be partially in contact, and bonding the contacted surfaces. The main surface refers to a surface having a much larger area than the other surfaces among the surfaces constituting the substrate (10, 20). The substrates (10, 20) each have two main surfaces, and these two main surfaces are positioned facing each other. The main surface 20a, which is partially in contact with the first substrate 10, has a recess. The recess is not shown in FIG. 1 because it is hidden in the view in FIG. 1; it is described with reference to FIGS. 2 and 3. Another main surface 20b of the second substrate is positioned at an opposite side from the first substrate 10, and includes liquid distribution ports (21, 22).

Hereinafter, in the state in which the first substrate 10 and the second substrate 20 are bonded together, the XYZ coordinate system is appropriately referred to such that the XY plane denotes the plane parallel to the main surface (10a, 10b) of the first substrate 10 and the main surface (20a, 20b) of the second substrate 20, and the Z direction denotes the direction orthogonal to the XY plane. In the case of FIG. 1, the liquid distribution ports (21, 22) are separated in the X direction. The liquid distribution ports (21, 22) are extended with respect to the Z direction so as to approach the main surface 20a of the second substrate 20; however their illustration is omitted in FIG. 1.

In the present specification, when the direction is expressed, a positive or negative sign is added to distinguish a positive direction from a negative direction, such as "X direction", "-X direction". In the case of expressing the direction without distinguishing a positive and negative direction, it is simply expressed as "X direction". In other words, in the present specification, the simple expression of "X direction" includes both of "+X direction" and "-X direction". This holds true for the Y direction and the Z direction.

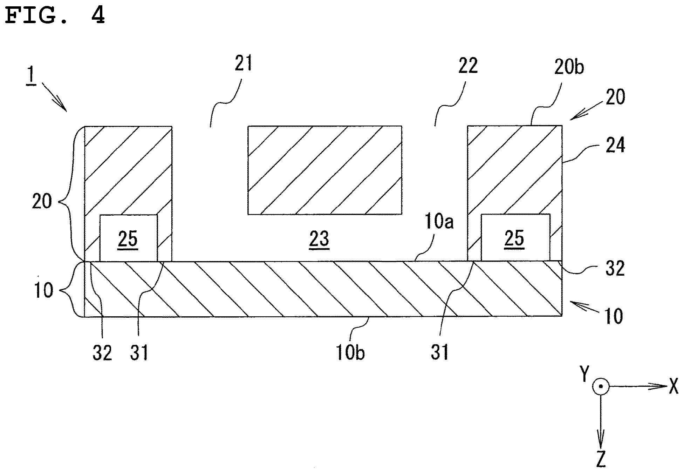

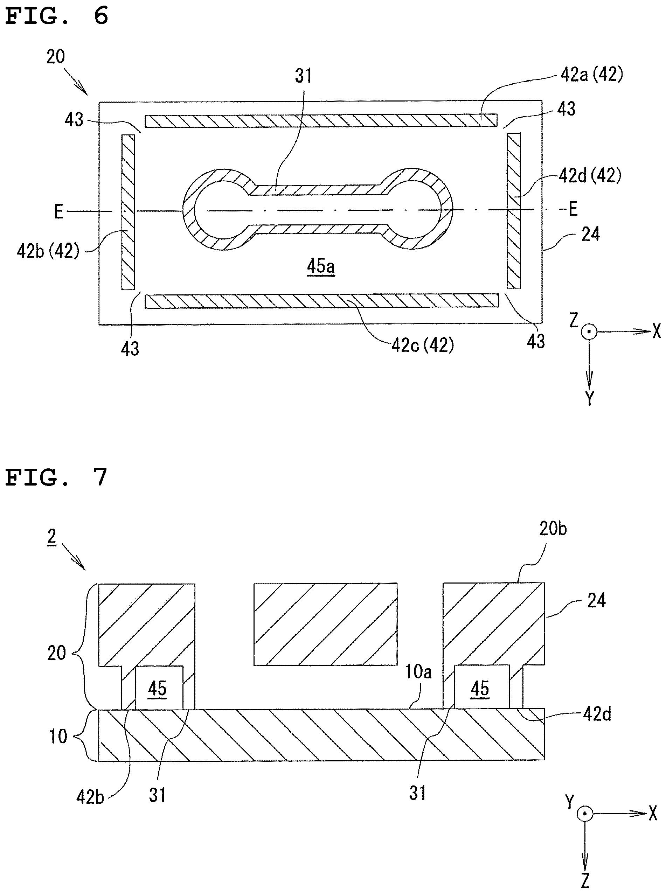

FIG. 2 is a plan view of the second substrate 20 before bonding when viewed from the side of the main surface 20a in the -Z direction. FIG. 3 is a schematic cross-sectional view of a microchip 1 in which the second substrate 20 is bonded on the first substrate 10; the cross-section is taken along line B-B of the second substrate in FIG. 2. FIG. 4 is a schematic cross-sectional view of the microchip 1 in which the second substrate 20 is bonded on the first substrate 10; the cross-section is taken along line C-C of the second substrate in FIG. 2. For ease of understanding, the schematic cross-sectional views in FIGS. 3 and 4 show only the lines that appear on the respective cross-sections of both substrates. The same holds true for FIG. 7, which will be described later.

A recess 23a provided on the main surface 20a functions as a hollow channel 23 sandwiched between the two substrates (10, 20) when the first substrate 10 are bonded to the second substrate 20. The liquid distribution ports (21, 22) are formed through the second substrate 20 from the channel 23 toward the main surface 20b of the second substrate. Hence, the hollow channel 23 communicates with the liquid distribution ports (21, 22).

Each of the liquid distribution ports (21, 22) has at least one of the following purposes: to inject liquid into the microchip 1, and to discharge liquid from the microchip 1. For example, the liquid distribution port 21 may be used as a liquid injection port and the liquid distribution port 22 may be used as a liquid discharge port. The state of the hollow channel 23 is not limited to a state in which the liquid flows when liquid is in the hollow channel; the state includes a state with no liquid flow, such as a state in which liquid is stored.

A bonding section includes a first bonding section 31 that is adjacent to the channel 23 and surrounds it, and a second bonding section 32 located closer to the side of an outer side surface 24 of the second substrate 20 than the first bonding section 31, when viewed from a direction orthogonal to the main surface 20b of the second substrate 20, in other words, the Z direction. With reference to FIG. 2, the first bonding section 31 corresponds to a hatching area shaded with upper-right diagonal lines, and the second bonding section 32 corresponds to a hatching area shaded with lower-right diagonal lines. The same holds true for FIGS. 6, 8, 9A, 9B, 10A and 10B, which will be described later. In the present specification, the outer side face refers to the outer surfaces of the substrates (10, 20) except the main surfaces thereof.

The first bonding section 31 serves to bond the two substrates (10, 20) and define the channel 23. The second bonding section 32 serves to reinforce the bonding strength of the two boards (10, 20), which is insufficient with only the first bonding section 31. The internal space 25 is formed between the first bonding section 31 and the second bonding section 32. In the present embodiment, a recess 25a provided on the main surface 20a of the second substrate 20, serves to form the internal space 25 at a position between the first bonding section 31 and the second bonding section 32 by bonding the main surface 10a of the first substrate 10 to the main surface 20a of the second substrate 20.

In the present embodiment, the second bonding section 32 has a line shape connecting a starting point 32s with an end point 32e that is different from the starting point 32s as shown in FIG. 2. Hence, there is a gap 33 between the starting point 32s and the end point 32e, where the second bonding section 32 does not exist. The internal space 25 formed with the recess 25a is communicated with the external space of the first substrate 10 and the second substrate 20, i.e., the external space of the microchip 1, through the gap 33. This configuration enables the effect of eliminating the pressure difference between the inside and outside of the microchip 1, which will be described below.

As shown in FIG. 2, the second bonding section 32 having a line shape according to the present embodiment is configured to be a polyline-shaped bonding section, which has a polyline shape, extending in contact with the outer side surface 24 of the second substrate 20. Since the second bonding section 32 is positioned at the outermost side of the second substrate 20, both of the substrates can be stably bonded. The bottom depth of the recess 25a (i.e., the length in the Z direction from the main surface (bonding surface) of the second substrate 20 to the bottom surface of the recess 25a) is preferably 0.05 mm or more and 3 mm or less.

The method of manufacturing the microchip 1 is described on the following.

### (Step S1: Preparation of substrate)

The first substrate 10 and the second substrate 20, which constitute the microchip 1, are prepared.

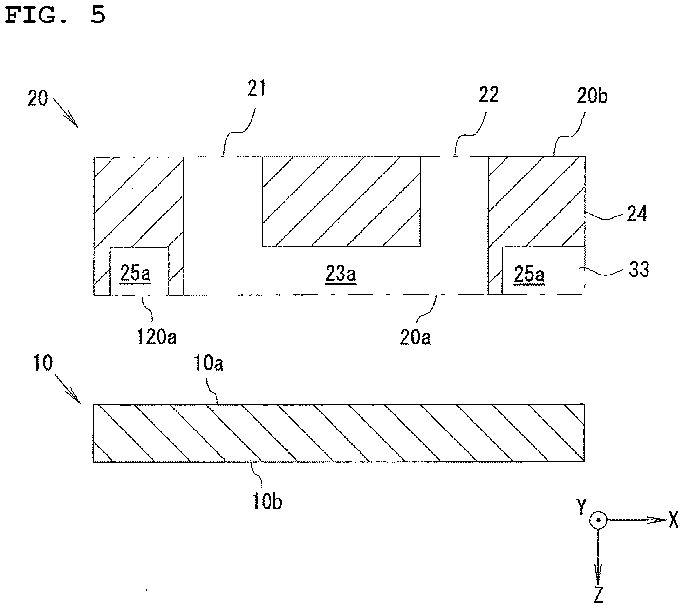

Fig. 5 is a schematic cross-sectional view of the first substrate 10 and the second substrate 20 shortly before bonding them in the manufacturing process of the microchip 1.

The material that constitutes the substrates (10, 20) is preferably a material of a substantially non-porous body. Here, "substantially non-porous body" means that the apparent surface area of the substrates approximates to the actual surface area. Examples of materials that form the non-porous body as described above are inorganic materials such as glass and silicon; and resin materials such as polymethyl methacrylate (PMMA), poly carbonate (PC), cyclo-olefin copolymer (COC), cyclo-olefin polymers (COP), polystyrene (PS), and silicone. Also two or more of these resin materials can be combined. It is also possible to use different materials for the first substrate 10 and the second substrate 20.

The shape of the substrates according to the present embodiment is described below. The first substrate 10 and the second substrate 20 are rectangular substrates having their main surfaces that are the same respective dimensions in length and width. The thickness of the second substrate 20 is greater than that of the first substrate 10. However, the first substrate 10 and the second substrate 20 may not have the same dimensions in length and width of the main surface. For example, the dimensions in length and width of the main surface of the first substrate 10 may be larger than the dimensions in length and width of the main surface of the second substrate 20; also the dimensions in length and width of the main surface of the second substrate 20 may be larger than the dimensions in length and width of the main surface of the first substrate 10. The thickness of the second substrate 20 may be the same as the thickness of the first substrate 10; the thickness of the second substrate 20 may be smaller than the thickness of the first substrate 10.

With reference to FIG. 5, both of the two main surfaces (10a, 10b) of the first substrate 10 according to the present invention are flat. The main surface 20a, which is one of the main surfaces of the second substrate 20, includes a portion used as the first bonding section 31 and second bonding sections 32, the recess 23a for forming the hollow channel 23 after being bonded to the first substrate 10, a recess 25a that defines the first bonding section 31 and the second bonding sections 32 to form an internal space 25. The end surfaces (bonding surfaces) of the second substrate 20 used as the first bonding section 31 and the second bonding section 32 are flat and can be bonded to the main surface 10a of the first substrate 10. The other main surface 20b of the second substrate 20 has openings that are later used as liquid distribution ports (21, 22). At least part of the recess 25a reaches the outer surface 24 of the second substrate.

Various methods are available to provide openings and recesses in the substrate (10, 20), for example, injection molding, combination of photolithography process and etching process, casting, and cutting and processing; however, the method can be appropriately selected according to the material constituting the substrates. For example, when the second substrate 20 is made of resin materials such as polymethyl methacrylate (PMMA), polycarbonate (PC), cyclo-olefin copolymer (COC), cyclo-olefin polymer (COP), polystyrene (PS), silicone, or acrylic described above, injection molding is used to easily form the recesses (23, 25). The first substrate 10 can also be made of glass materials such as borosilicate glass in addition to the above resin material, if no recesses are provided.

### (Step S2: Bonding of substrate)

The main surface 10a of the first substrate 10 fabricated in step S1 is bonded to the main surface 20a of the second substrate 20. The bonding method described below is a method that eliminates the need for forming a thin film of adhesive on the substrate. It is performed in the following steps.

First, the bonding surfaces (10a, 20a) of both substrates are treated to activate the surface. The method of surface activation treatment includes irradiation with ultraviolet light and contact with plasma gas.

The method of ultraviolet irradiation is performed by irradiating the main surface 20a of the second substrate 20 and the main surface 10a of the first substrate 10 with vacuum ultraviolet light, which has a wavelength of 200nm or less, emitting from an ultraviolet light source, for example, a xenon excimer lamp having an emission line at a wavelength of 172 nm. Other examples of ultraviolet light sources include low-pressure mercury lamps having an emission line at a wavelength of 185 nm and deuterium lamps having an emission line in a wavelength range of 120-200 nm. The irradiance of the vacuum ultraviolet light is, for example, 10-500 mW/cm2, and the irradiation time is appropriately set according to the resin; however, it is, for example, 5-6 seconds.

The method of contacting the plasma gas is performed by contacting the main surface 20a of the second substrate 20 and the main surface 10a of the first substrate 10 with a process gas being composed mainly of nitrogen gas or argon gas with 0.01 to 5% oxygen gas by volume, and being converted into plasma with atmospheric pressure plasma. A mixture of nitrogen gas and clean dry air (CDA) can also be used. The contact time of the plasma gas is, for example, 5 to 100 seconds.

Next, the second substrate 20 is superimposed on the first substrate 10 so as to be in contact with both of the bonding surfaces (10a, 20a), on which the surface activation treatment have been performed. The two substrates are pressed using press machines to perform a bonding process. In order to maintain the surface activation state, the bonding process may be preferably performed within a predetermined time, for example, within ten minutes, after the completion of the UV irradiation process.

This bonding process is performed under heated conditions, if necessary, to reinforce the bonding strength. In the bonding process, the bonding conditions such as heating temperature and pressing force are set according to the constituent material of the first substrate 10 and the constituent material of the second substrate 20. Examples of the specific conditions are that the temperature during the pressing is 40-130°C and the pressing force for bonding is 0.1-10 MPa.

A substrate formed by bonding the first substrate 10 and the second substrate 20 (hereinafter referred to as a "bonded substrate") may be heated for a further predetermined time as necessary after being pressurized for a predetermined time. Even when the bonded substrate has a mixture of an area where a sufficient bonding state has been obtained and an area where an insufficient bonding state has been obtained at a bonding interface between the stacked substrate after the pressurization, heating the bonded substrate enables the area where the insufficient bonding state has been obtained to have a desired state.

The pressurized state of the bonded substrate may be maintained for a predetermined time, then the pressurized state may be released and the temperature of the bonded substrate may be raised to a predetermined temperature and maintained until the desired bonding state is obtained. Here, the predetermined temperature refers to the temperature at which deformation does not occur in the bonded substrate. For example, the heating temperature is 40-130°C and the heating time is 60-600 seconds.

Through the cooling process, the microchip 1 in which the second substrate 20 has been bonded on one main surface of the first substrate 10 is fabricated.

Here, as described above, the recess 25a reaches at least part of the outer surface 24 of the second substrate 20. Hence, by forming the first bonding section 31 and the second bonding section 32 through the bonding process in the present step S2, the recess 25a forms the internal space 25 sandwiched between both of the bonding sections (31, 32); however, the internal space 25 is communicated with the external space of the substrates (10, 20). Therefore, even if the microchip is heated in the present step S2, there will not be a large difference in pressure between the pressure in the internal space 25 and the pressure of the external space. As a result, the bonding strength between the first substrate 10 and the second substrate 20 is improved, compared with the microchip 200 shown in FIG. 12A. Furthermore, since the internal space 25 is not likely to become negative pressure even after cooling, the liquid stored in the channel 23 is suppressed from flowing out over the first bonding section 31 into the internal space 25 when using.

As an exemplary modification, the main surface 10a of the first substrate 10 may also be provided with a recess to form a hollow channel or a recess to define the first bonding section 31 and the second bonding section 32. Moreover, the main surface 10b of the first substrate 10, which is not bonded to the second substrate 20, may also have openings formed thereon to be used as liquid distribution ports (21, 22).

In addition, in the present embodiment, the second bonding section 32 is described as exhibiting a polyline shape that extends in contact with the outer side surface 24 of the second substrate 20; however, it may be a polyline shape located at a position outside the first bonding section 31 and inside the outer side surface 24. In this case as well, a gap 33 for communicating between the external space and the internal space 25 may be formed at least at part of the second bonding section 32. The second bonding section 32 is not limited to a polyline shape; it may be a curve shape.

In FIG. 2, the distance between the starting point 32s and the ending point 32e in the Y direction, i.e., the length d of the gap 33 in the Y direction, is preferably 0.05 mm or more and 50 mm or less. In addition, when the length d in the Y direction is 0.1 mm or less, it is possible to prevent the entry of foreign matters as a secondary effect of providing the second bonding section 32. Also, in FIG 2, the second bonding section 32 has only one gap, however, it may have a plurality of gaps as an exemplary modification. FIG. 6 described below can be considered as an example of the polyline-shaped second bonding section 32 having a plurality of gaps.

In the example shown in FIG. 2, the second bonding section 32 includes polyline-shaped portions provided with angular portions at the corners of the second substrate 20 when viewed from the Z direction; however the polyline-shaped portions may be chamfered at the vicinity of the corners. If the corner of the second bonding section 32 is angular, the polyline-shaped portion is prone to be distorted by stress concentration associated with pressing force when bonding the substrates (10, 20). Distortion of the bonding section lowers the bonding strength. The chamfered corner of the polyline-shaped portion alleviates the stress concentration, suppresses the distortion, thereby improving the bonding strength.

### [Second Embodiment]

The second embodiment of the microchip according to the present invention will be described with reference to FIGS. 6 and 7. The items described in the first embodiment can be implemented to the second embodiment in the same manner except as described below. The same applies to the third and subsequent embodiments.

FIG. 6 is a plan view of a second substrate 20 before bonding according to the microchip 2 of the present embodiment (See FIG. 7) when viewed from the main surface of the second substrate 20 in the -Z direction, the surface to be bonded with the first substrate 10. FIG. 7 is a schematic cross-sectional view of a microchip 2 that the second substrate 20 of the present embodiment is bonded to the first substrate 10. The cross section of the view is taken along line E-E of the second substrate 20 in FIG. 6.

The second bonding sections 42 are located closer to the outer side surfaces 24 of the second substrate 20 than the first bonding section 31. The second bonding sections 42 include 4 line segment bonding sections (42a to 42d) configured to be lines inside the outer side surfaces 24, without being contact with the outer side surfaces 24, and extending along the outer side surfaces 24 when viewed from the Z direction. In the present embodiment, the four outer side surfaces 24 of the rectangle second substrate 20 each have the respective line segment bonding sections (42a to 42d) extending along the outer side faces. The line segment bonding sections do not intersect each other and have a gap 43 between the line segment bonding sections. A recess 45a of the second substrate that is located between the first bonding section 31 and the line segment bonding sections (42a to 42d) forms an internal space 45 after bonding the second substrate 20 to the first substrate 10. The internal space 45 is communicated with the external space of the first substrate 10 and the second substrate 20, i.e., the external space of the microchip 2, via a plurality of the gaps 43.

In the present embodiment, the gaps 43 are provided in the vicinity of all corners of the second substrate 20. Hence, the second bonding sections 42 each having a line shape do not need to have a corner, resulting in alleviating the stress concentration during the pressing of the substrates. The line edges of the second bonding sections 42 may be rounded to further alleviate the stress concentration.

In addition, in the present embodiment, the four outer side surfaces 24 each have one respective line segment bonding sections (42a to 42d) extending parallel to the outer side faces; however, each outer side face may have two or more line segment bonding sections. The second bonding sections 42 each may not be a line shape when viewed from a direction orthogonal to the main surface of the second substrate; it may be a curve shape.

The gap 43 in the XY plane is preferably 0.05 mm or more and 50 mm or less. The gap 43 may preferably be 0.1 mm or less to achieve the effectiveness in preventing foreign matters from entering as a secondary effect.

In addition, the line segment bonding sections (42a to 42d) each constituting the second bonding section 42 may be parallel to the respective outer side surfaces 24 (i.e., sides), or may not be parallel thereto viewed from the Z direction. In other words, the second bonding section 42 is considered to be within the scope of the configuration of the present embodiment as long as the second bonding section 42 includes a plurality of line segment bonding sections located at closer to the outer side (outer side faces 24) than the first bonding sections 31 when viewed from the Z direction, the line segment bonding sections being arranged in the XY plain and separated via the gaps 43.

### [Third Embodiment]

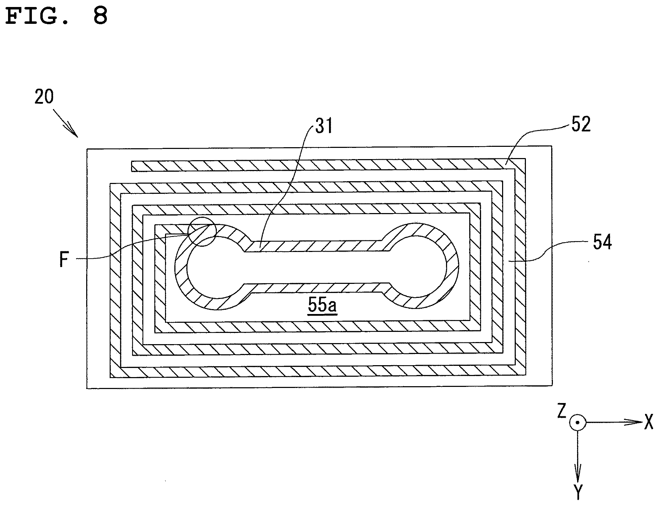

The third embodiment of the microchip according to the present invention is described with reference to FIG. 8. In the microchip according to the present embodiment, FIG. 8 is a plan view of a second substrate 20 before bonding when viewed from the main surface thereof, the surface to which the first substrate 10 is to be bonded.

The second bonding section 52 of the second substrate 20 is configured to be a spiral bonding section that is formed in a spiral shape and at a location at which its center locates the first bonding section when viewed from a direction orthogonal to the main surface of the second substrate 20 (i.e., Z direction). The spiral bonding section includes a communicating channel 54 that has a spiral shape and communicates an internal space that is formed by the recess 55a enclosed by the first bonding section 31 and the second bonding section 52 that is closest to the first bonding section 31, with the external space of the first substrate 10 and the second substrate 20, in other words, the external space of the microchip. Forming the communicating channel 54 in a spiral shape enables the channel length to be longer. Hence, this configuration provides an effect of decreasing the pressure difference between the internal space and the external space when manufacturing the microchip, and also enhances the effectiveness of preventing the entry of foreign matters as a secondary effect.

With reference to FIG. 8, the second bonding section 52 is connected to the first bonding section 31 at F portion when viewed from Z direction. In this way, even when the second bonding section 52 is connected to the first bonding section 31, this configuration allows the internal space formed with the recess 55a enclosed by the first bonding section 31 and the second bonding section 52 to be communicated with the external space of the first bonding substrate 10 and the second bonding substrate 20. It is noted that the second bonding section 52 may be formed to be apart from the first bonding section 31 on the XY plain.

### [Fourth Embodiment]

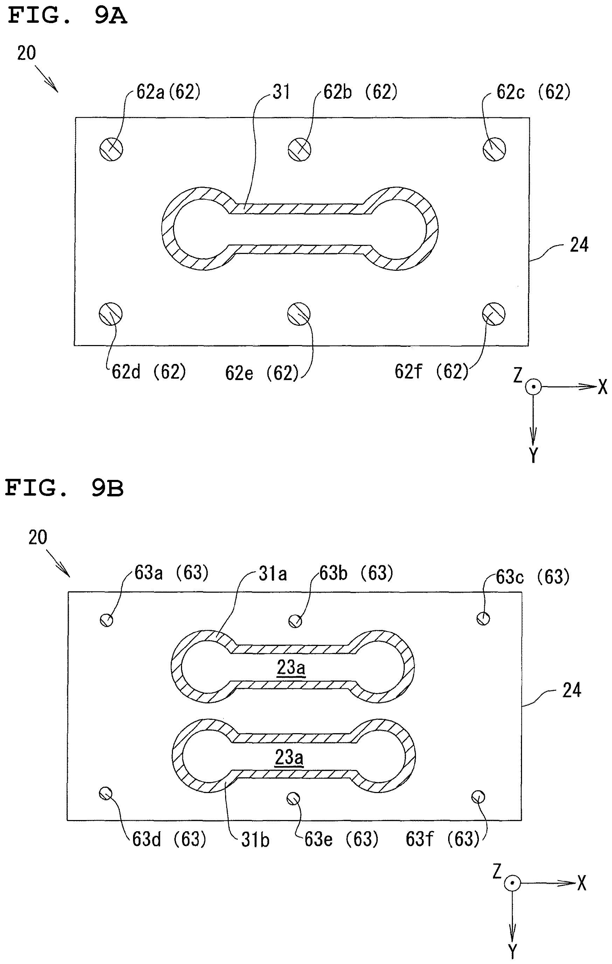

With reference to FIG. 9, the fourth embodiment of the microchip according to the present invention will be described. FIG. 9A is a plan view of a second substrate 20 before bonding according to the microchip of the present embodiment when viewed from the Z direction. The second bonding section 62 of the second substrate 20 includes local bonding sections (62a-62f) that are discretely arranged in a plurality of positions on the XY plain when viewed from a direction orthogonal to the main surface of the second substrate 20 (i.e., Z direction).

In the present embodiment, the second bonding section 62 is composed of six local bonding sections (62a-62f), each of the local bonding sections are a circular shape when viewed from Z direction. The space enclosed by the local bonding sections (62a-62f) and the first bonding section 31 communicates with the external space of the first substrate 10 and the second substrate 20. The second bonding section 32, as described above, serves to reinforce the bonding strength of the two boards (10, 20), which is insufficient with only the first bonding section 31. By arranging the second bonding sections 32 in a discrete manner, it is possible to achieve uniform bonding strength over the entire surface of the substrate.

Each of the local bonding sections (62a-62f) constituting the second bonding section 62 is disposed locally at the outside of the first bonding section 31 (the outer side faces 24 of the second substrate 20). The local bonding sections (62a-62f) each can be any shape of, for example, elliptical, polygonal, or linear, which is other than circular. The local bonding sections (62a-62f) may also have a smaller area than the first bonding section 31.

FIG. 9B is a plan view of a second substrate 20 according to the exemplary modification of the present embodiment when viewed from the main surface to which the first substrate is to be bonded.

FIG. 9B illustrates the second substrate 20 that includes two recesses 23a to form two channels. The second substrate 20 includes two first bonding sections (31a, 31b) that surround the respective recesses 23a, and second bonding sections 63 outside (outer side faces 24 of the second substrate 20) the two bonding sections (31a, 31b). The sum of the bonding areas of the first bonding sections 31 is larger than the sum of the bonding area of the first bonding section 31 in FIG. 9A.

The size of each of the local bonding sections (63a-63f) is determined such that the sum of each of the bonding areas of local bonding sections (63a-63f) constituting the second bonding section 63 is smaller than the sum of each of the bonding areas of local bonding sections (62a-62f) constituting the second bonding section 62 in FIG. 9A. This enables the sizes of local bonding sections (63a-63f) to be determined such that the sum of the bonding area of the first bonding section 31 and the bonding area of the second section 62 accounts for a desired range (for example, 30% or less with respect to the total area of the main face of the second substrate).

When the sum of the bonding area of the first bonding section 31 and the bonding area of the second bonding section 62 is within a desired numerical range, this makes it possible to obtain an effect that no processing conditions of the press machine (e.g., pressing force, pressing time, and heating temperature) is necessary to be modified. In other words, when a press machine is used to bond different shapes of substrates continuously, if the bonding areas of the substrates to be successively bonded are all within the desired numerical range, it is possible to eliminate the need for modifying the settings of the processing conditions of the press machine for each substrate.

The local bonding sections constituting the second bonding sections (62, 63) may not all be of the same size or shape. The size of each of the local bonding sections may be set to positively correlate with the length of the distance from the nearest first connection 31. In other words, local bonding sections located relatively close to the first bonding section 31 may be made smaller, and local bonding sections located relatively far from the first bonding section 31 may be made larger. The size of the bonding sections may be set such that a ratio of the area of the bonding sections to the unit area of the second substrate 20 (i.e., a ratio of the sum of the area of the first bonding section 31 and the area of the second bonding sections (62, 63) to the area of the main face of the second substrate 20) is constant. This setting enables the local bonding strength of the microchip to be uniform. The shape and arrangement of the local bonding sections may be set based on the distance to the first bonding section 31, and the shape and orientation of the first bonding section 31.

### [Fifth Embodiment]

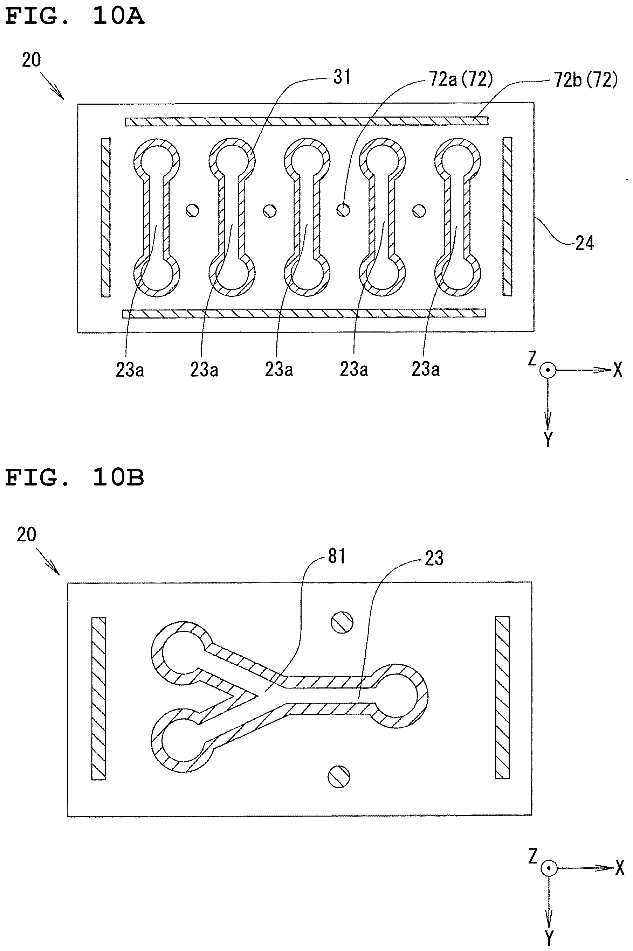

An example of combining the above embodiments is described as the fifth embodiment. FIG. 10A is a plan view of a second substrate 20 of the microchip according to the present embodiment before bonding when viewed from the main surface thereof on which the first substrate 10 is to be bonded. The second substrate 20 shown in FIG. 10A includes five recesses 23a each extending in the Y direction and being separated apart in the X direction for forming the channel, the first bonding sections 31 each being adjacent to surround the respective recesses 23a, and second bonding sections 72. The second bonding sections 72 includes local bonding sections 72a located between the adjacent first bonding sections 31 and line segment bonding sections 72b each being locate at the outer side of the all recesses 23a of the second substrate and extending along the respective outer side faces 24. The second bonding sections 72 may have their shape, size (length and width), number and arrangement such that the ratio of the area of the bonding section to the unit area of the substrate is constant.

Although cell and tissue culturing is described as an application of the microchips, the microchips according to the present invention can find their use for purposes other than cell and tissue culturing; for example, the microchips according to the present invention can be used for various applications such as mixing, separation, reaction, synthesis, extraction, or analysis of small amount of fluid (not limited to liquid).

As an example, FIG. 10B shows a plan view of the second substrate 20 used in a microchip having a branching channel 81 in the middle. The microchip shown in FIG. 10B includes three liquid distribution ports, two of which are used for injecting liquid. The branching channel 81 is suitable for mixing, reaction and synthesis of multiple fluids, separation into multiple fluids or the like.

In addition, all liquid distribution ports may be used for liquid injection ports when no liquid inside the microchips is necessary to be exhausted in the case, for example, in which microchips are used for analysis. One liquid distribution port may be provided at each channel.

This invention is not limited to the embodiments described above, and various improvements and modifications can be made without departing from the scope of the present invention.

### REFERENCE SIGNS LIST

- 1,2,100,200: Microchip
- 10,110: First substrate
- 20, 120: Second substrate
- 21, 22, 121, 122: Liquid distribution port
- 23,123: Channel
- 24, 124: Outer side face
- 23a, 25a, 45a, 55a, 123a, 250a: Recess
- 25, 45, 250: Internal space
- 31, 131: First bonding section
- 32, 42, 52, 62, 72, 132: Second bonding section
- 33,43: Gap

## Claims

1. A microchip comprising:
a first substrate;
a second substrate that is partially bonded to the first substrate;
a hollow channel that is located between the first substrate and the second substrate, the channel extending in a direction along a main surface of the second substrate;
a liquid distribution port that is formed to penetrate the second substrate from the channel toward the main surface thereof located at an opposite side of the first substrate;
a first bonding section that bonds the first substrate to the second substrate to surround the channel when viewed from a direction orthogonal to the main surface;
a second bonding section located at a position closer to an outer side face of the second substrate than the first bonding section, and that bonds the first substrate to the second substrate; and
an internal space provided between the first bonding section and the second bonding section, and that communicates with a space outside the first substrate and the second substrate.

2. The microchip according to the claim 1, wherein the second bonding section includes a portion that has a line shape connecting a starting point with an end point that is different from the starting point when viewed from a direction orthogonal to the main surface.

3. The microchip according to the claim 2, wherein the second bonding section includes a bonding section that has a polyline shape extending with being in contact with the outer side face of the second substrate when viewed from a direction orthogonal to the main surface.

4. The microchip according to any one of the claims 1 to 3, wherein the second bonding section includes a plurality of line segment bonding sections that are composed of line segments extending along the outer side faces of the second substrate without being in contact with the outer side faces thereof when viewed from a direction orthogonal to the main surface, the line segment bonding sections being formed apart from each other.

5. The microchip according to any one of the claims 1 to 4, wherein the second bonding section includes a spiral bonding section that is formed such that its center is located at the first bonding section when viewed from a direction orthogonal to the main surface.

6. The microchip according to any one of the claims 1 to 5, wherein the second bonding section includes local bonding sections that are disposed discretely at a plurality of locations when viewed from a direction orthogonal to the main surface.

7. The microchip according to claim 6, wherein the local bonding sections each are a circular shape, an elliptical shape or a polygonal shape when viewed from a direction orthogonal to the main surface.

8. The microchip according to any one of claims 1 to 7, wherein a part of the second bonding section is connected to the first bonding section when viewed from a direction orthogonal to the main surface.
